# EUROPEAN PATENT SPECIFICATION

(11) **EP 4 512 444 B1**
(45) Date of publication and mention of the grant of the patent: **29.04.2026**
(21) Application number: 23802816.1
(22) Date of filing: 06.05.2023
(51) Int. Cl.: A61M 5/178, A61M 5/32

(54) **NEEDLE PROTECTION TYPE PRE-FILLED BARREL, ASSEMBLY COMPRISING SAME AND MOUNTING METHOD FOR ASSEMBLY**
VORGEFÜLLTE NADELSCHUTZTROMMEL, ANORDNUNG DAMIT UND MONTAGEVERFAHREN FÜR ANORDNUNG
CYLINDRE PRÉ-REMPLI DE TYPE À PROTECTION D'AIGUILLE, ENSEMBLE LE COMPRENANT ET PROCÉDÉ DE MONTAGE POUR L'ENSEMBLE

(30) Priority: 07.05.2022 CN 202210492992
(43) Date of publication of application: 26.02.2025
(73) Proprietor: Shanghai Innopac Medical Technology Co., Ltd., Shanghai 201605 (CN)
(72) Inventor: GUO, Rong, Shanghai 201605 (CN)
(74) Representative: Garavelli, Paolo
(86) International application number: PCT/CN2023/092562
(87) International publication number: WO 2023/217044

(56) References cited:
- WO-A1-2015/114318
- CH-A2- 714 526
- CN-A- 110 201 271
- CN-A- 114 642 792
- CN-A- 114 642 793
- CN-A- 114 652 920
- CN-A- 114 733 007
- CN-A- 114 796 727
- CN-A- 114 796 728
- CN-U- 217 593 519
- CN-U- 218 220 747
- CN-U- 218 636 425
- CN-U- 218 636 426
- US-A1- 2016 228 654
- US-A1- 2019 015 599
- US-B1- 6 527 742

## Description

### TECHNICAL FIELD

The present disclosure belongs to the technical field of medical appliances, and particularly relates to a needle protection type pre-filled barrel and an assembly thereof, and a mounting method for the assembly.

### BACKGROUND ART

A syringe is a medical apparatus used for injecting liquid medicine into human bodies or used for cleaning, and is often provided with a needle when used for injection for the human bodies. Because the syringe needs to be filled with medicine before injection, in a traditional method of filling the syringe with medicine, a needle tip is inserted into a medicine bottle to draw liquid medicine, however, time and labor and medicine are wasted in this way. Therefore, a pre-filled syringe is available on the market at present, and the syringe is filled with medicine in advance through a fill-finish device, and then the syringe has been filled with medicine during actual use, thereby saving time and labor during use. For example, Chinese Utility Model Patent No. CN213191809U, published on May 14, 2021, discloses a pre-filled syringe. The syringe needle (7) is covered by a cap body (1). During use, the cap body (1) is removed to expose the syringe needle (7) for injection. However, after such use, the syringe needle (7) is typically discarded in an exposed state. Furthermore, Chinese Utility Model Patent No. CN206350852U, published on July 25, 2017, discloses another pre-filled syringe. This syringe includes an injection needle (2) located at the front end of a glass syringe barrel and a needle cap (1) used to protect the injection needle (2). Similarly, after this pre-filled syringe is used, the injection needle (2) is discarded in an exposed state.

To mitigate the problem of needle exposure after discarding the pre-filled syringe, a common solution is to recap a previously used protective cover(such as the needle cap described in CN206350852U) over the needle to prevent needle exposure. However, this approach has several defects: firstly, the protective cover, serving as a temporary protector, is prone to detachment from the needle, with an even higher likelihood of detachment occurring during the rough handling of waste transportation; secondly, the protective cover typically sleeves the needle snugly during the automated manufacturing process in factories, which has a very small diameter, whereas the manual method of recapping the protective cover over the needle is not only energy-draining and detrimental to the efficiency of nurses but also poses a high risk of needlestick injuries to fingers of the nurses if the protective cover is not aligned properly with the needle during the recapping process, potentially causing the nurses to be exposed to infectious diseases from patients; and thirdly, the protective cover is generally designed as a single-use component that is often discarded after injection, making the protective cover difficult to locate after injection. Therefore, the method relying on recapping the protective cover over the needle to prevent needle exposure is unreliable.

To solve the above problem, the market has introduced a protective cover structure specifically designed for accommodating the needle, such as the Chinese utility model patent with Publication Number CN215083511U, published on December 10, 2021, which has disclosed a detachable needle protective sleeve. This protective sleeve includes a cartridge that sleeves a needle protection cap and a protective head that engages with an end of the needle protection cap and is connected to the cartridge, and both the cartridge and the protective head are integrally formed; an engaging groove matched with the end of the needle protection cap is formed in an inner side of the protective head; and both a longitudinal section of the cartridge and a longitudinal section of the protective head are in the shape of a regular polygon, and cross-sectional areas at junctures of the cartridge and the protective head are identical. By sleeving the needle protection cap with the cartridge, any person using the syringe can be protected without altering the existing specifications of the syringe, and meanwhile, the protective head serves to safeguard fingers from needlestick injuries during use.

However, this utility model further has the following defects: firstly, the detachable protective sleeve design, no matter how sophisticated the structure is, inherently carries the risk of detachment from the syringe during waste transportation; and secondly, the protective head used to sleeve the needle in the utility model requires alignment with the needle for mounting, which still imposes an additional workload of nurses, with the risk of needlestick injury to the nurses. WO 2015/114318 A1 and US 6 527 742 B1 disclose further protective cover structures designed for accommodating the needle.

Therefore, in conclusion, in order to meet market requirements, a pre-filled needle with a protective cover is urgently needed at present, in which the protective cover is mounted with the barrel during the preparation of products and can stably cover the needle after the syringe is used.

### SUMMARY

The present disclosure aims at providing a needle protection type pre-filled barrel and an assembly thereof, and a mounting method for the assembly. The technical effect that a needle can be stably covered after a syringe is used is achieved through a limiting block unit located on a needle mounting tube of the barrel and a protective cover unit sleeving the needle mounting tube and covering an injection needle.

A technical solution of the present disclosure to solve the above problems is as follows: a needle protection type pre-filled barrel, includes: a barrel, a needle mounting tube, an injection needle, and a sealing sleeve, wherein
the needle mounting pipe is arranged on the barrel and used for mounting the injection needle, the barrel, the needle mounting tube, and the injection needle are in a connected state, the sealing sleeve sleeves the needle mounting tube and is used for protecting the injection needle, and the needle protection type pre-filled barrel further includes:
a limiting block unit, including limiting blocks mounted on the needle mounting tube; and
a protective cover unit, including a protective cover sleeving part of the barrel and all the needle mounting tube and injection needle, limiting ports being formed in the protective cover and used for engaging with the limiting blocks.

The protective cover unit further includes:
an energy arm portion, including an energy arm with one end arranged on inner side faces of distal ends of the limiting ports and the other end with an elastic force directed inward in a radial direction of the protective cover.

A straight surface for locking is arranged at a proximal end of the energy arm and used for jacking the limiting blocks so as to lock relative positions between the protective cover and the injection needle.

The energy arm portion further includes a jacking block arranged at the proximal end of the energy arm, and an inclined surface for unlocking is arranged at a proximal end of the jacking block.

When the sealing sleeve sleeves the needle mounting tube, the energy arm or the jacking block is jacked outward in a radial direction of the barrel, such that the straight surface for locking and the limiting blocks disengage from each other.

The needle protection type pre-filled syringe further includes a rotating limiting unit, wherein the rotating limiting unit includes a rotating ring sleeving the needle mounting tube and guide blocks arranged on an outer ring surface of the rotating ring, and the guide blocks are provided with distal inclined surfaces and proximal inclined surfaces;
the protective cover unit further includes a guide groove portion, and the guide groove portion includes:
an inlet groove formed in an inner ring surface of the protective cover and used for mounting the guide blocks;
a first guide surface located at a distal end of the inlet groove, wherein when the protective cover moves toward a proximal end, the first guide surface and the distal inclined surfaces slide in a fit manner, so as to drive the rotating ring to rotate;
a second guide surface connected with the first guide surface and used for jacking the guide blocks to prevent the rotating ring from rotating;
a third guide surface arranged on the inner ring surface of the protective cover and located at a proximal end of the second guide surface, wherein when the protective cover moves to a distal end, the third guide surface and the proximal inclined surfaces slide in a fit manner, so as to drive the rotating ring to rotate;
a fourth guide surface connected with the third guide surface and used for jacking the guide blocks to prevent the rotating ring from rotating; and
a fifth guide surface arranged on the inner ring surface of the protective cover and located at a distal end of the third guide surface, wherein when the protective cover moves to the proximal end, the fifth guide surface and the distal inclined surfaces slide in a fit manner, so as to drive the rotating ring to rotate to enter a withdrawal groove formed in the inner ring surface of the protective cover.

The rotating limiting unit further includes energy arm jacking blocks arranged on the rotating ring, and when the guide blocks are located in the inlet groove, the energy arm jacking blocks jack the energy arm or the jacking block outward in the radial direction of the protective cover, such that the straight surface for locking and the limiting blocks disengage from each other.

In a further preferred technical solution, the protective cover unit further includes a window for demolding that is arranged in the protective cover, and the window for demolding exposes a position of the guide groove portion in the inner ring surface of the protective cover.

In a further preferred technical solution, the protective cover unit further includes inclined surfaces for mounting arranged at the proximal end of the protective cover and used for jacking the limiting blocks when the protective cover sleeves the needle mounting tube, such that the proximal end of the protective cover expands outward in the radial direction.

In a further preferred technical solution, the protection cover unit further includes a deformation groove that is formed in the proximal end of the protective cover and provides an expansion space when the proximal end of the protective cover expands outward in the radial direction.

In a further preferred technical solution, a display surface for printing patterns is arranged on an outer side face of the energy arm.

In a further preferred technical solution, the protective cover unit further includes an indicating member arranged on an outer side face of the protective cover.

In a further preferred technical solution, the rotating ring consists of a large rotating ring and a small rotating ring, and when the rotating ring sleeves the needle mounting tube, the large rotating ring is located at the proximal end, and the small rotating ring is located at the distal end.

In a further preferred technical solution, the limiting block unit further includes a mounting ring mounted on the needle mounting tube in a sleeving manner, and the limiting blocks are mounted on the needle mounting tube by being arranged on the mounting ring.

In a further preferred technical solution, a anti-rotation outer ring surface is arranged on the needle mounting tube, and a anti-rotation inner ring surface matched with the anti-rotation outer ring surface is arranged on an inner ring surface of the mounting ring.

In a further preferred technical solution, the needle mounting tube further includes a stop protrusion used for engaging with the rotating ring.

An assembly of a needle protection type pre-filled needle includes a push rod and a plunger, wherein
the push rod is arranged on the plunger; and
the plunger is mounted in a barrel and used for pushing out liquid medicine through an injection needle when pushing the push rod toward a distal end.

A mounting method for an assembly, includes the following steps:
S1, axially mounting a sealing sleeve into a protective cover;
S2, axially sleeving a needle mounting tube with the protective cover with the sealing sleeve;
S3, pushing a plunger into a barrel; and
S4, arranging a push rod on the plunger.

A use method for an assembly, includes the following steps:
S1, axially moving a protective cover along a proximal end of a barrel, such that a sealing sleeve is exposed;
S2, unlocking the sealing sleeve, to allow an injection needle to be exposed;
S3, carrying out injection; and
S4, axially moving the protective cover along a distal end of the barrel, such that the injection needle is completely covered.

The present disclosure has the following advantages:
firstly, the protective cover and the barrel are designed as an integrated unit, offering the advantage of preventing detachment. Once the protective cover fully covers and locks the used needle, it is nearly impossible for the needle to be exposed again;
secondly, during use, an operator only needs to pull the protective cover toward the center of the palm with the thumb and the index finger to expose the sealing sleeve and release the protective cover to lock the protective cover in place, which is conducive to injection;
thirdly, after injection, the operator pulls the protective cover toward the center of the palm again to reset the protective cover in an ejecting manner, and then a used syringe is covered with the protective cover, such that the syringe is convenient to discard;
fourthly, after the protective cover is reset and locked, a "click" sound will be given to remind the operator that the protective cover has been locked;
fifthly, in the presence of the sealing sleeve, the energy arm is naturally jacked, and the protective cover is not in an engaged state, such that the syringe is convenient to use by the operator; and
sixthly, the energy arm jacking blocks are arranged on the rotating ring with the guide blocks, the energy arm is jacked before use of the syringe by means of rotation of the rotating ring, the protective cover is not in the engaged state accordingly, and the protective cover disengages from the energy arm after use of the syringe, such that the protective cover restores to the engaged state.

### BRIEF DESCRIPTION OF THE DRAWINGS

FIG. 1 is an exploded diagram of a pre-filled barrel according to the present disclosure.
FIG. 2 is a schematic structural diagram illustrating a protective cover according to the present disclosure at a distal end of a barrel when a sealing sleeve is not removed.
FIG. 3 is a schematic diagram illustrating a structure with a sealing sleeve not removed according to the present disclosure.
FIG. 4 is a schematic diagram illustrating a structure with a sealing sleeve removed according to the present disclosure.
FIG. 5 is a schematic structural diagram illustrating a protective cover according to the present disclosure at a distal end of a barrel when a sealing sleeve is removed.
FIG. 6 is a schematic cross-sectional view taken along a line AA of FIG. 2.
FIG. 7 is an enlarged schematic view of FIG. 6.
FIG. 8 is an enlarged schematic view of FIG. 7.
FIG. 9 is a schematic structural diagram illustrating a protective cover unit according to the present disclosure.
FIG. 10 is a structural schematic diagram illustrating a guide groove portion according to the present disclosure.
FIG. 11 is a schematic structural diagram of a rotating limiting unit according to the present disclosure.
FIG. 12 is a schematic structural diagram of a limiting block unit according to the present disclosure.
FIG. 13 is a schematic structural diagram illustrating a limiting block unit sleeving a needle mounting tube according to the present disclosure.

In the drawings, the reference numerals denote the following components: limiting block unit 1, protective cover unit 2, rotating limiting unit 3, barrel 11, needle mounting tube 12, injection needle 13, sealing sleeve 14, mounting ring 101, limiting block 102, anti-rotation inner ring surface 103, protective cover 201, limiting port 202, energy arm portion 203, guide groove portion 204, window for demolding 205, deformation groove 206, inclined surface 207 for mounting, indicating member 208, rotating ring 301, guide block 302, energy arm jacking block 303, energy arm 203a, straight surface 203b for locking, jacking block 203c, inclined surface 203d for unlocking, inlet groove 204a, first guide surface 204b, second guide surface 204c, third guide surface 204d, fourth guide surface 204e, fifth guide surface 204f, withdrawal groove 204g, large rotating ring 301a, small rotating ring 301b, distal inclined surface 302a, proximal inclined surface 302b, anti-rotation outer ring surface 12a, stop protrusion 12b, and sealing sleeve protrusion 14a.

### DETAILED DESCRIPTION OF THE EMBODIMENTS

Definitions
1. Arrangement: It refers to the manner in which welding, riveting, threaded connection, screwing and the like are used to connect two components together.
2. Inner: It refers to one side closer to a narrower area or farther from a more open area. Similarly, "outer" refers to one side closer to a more open area or farther from a narrower area.
3. Engagement: It refers to a method by which a component A temporarily is secured to a component B by abutting against an inner and/or outer side surface of the component B.
4. Distal end: It refers to one end of a component mounted on a barrel that is farther from an opening where the barrel is provided with a push rod or closer to a mounting position of an injection needle. Similarly, a "proximal end" refers to one end of the component mounted on the barrel that is closer to the opening where the barrel is provided with the push rod or farther from the mounting position of the injection needle.
5. Axial direction: It refers to a direction where a straight line is formed by extending infinitely in a direction of the injection needle.
6. Radial direction: It refers to a linear direction along a diameter or radius, or a linear direction perpendicular to an axis.
7. Jacking: It refers to an action of applying a force by a first component onto a second component, so that the second component moves toward a direction opposite to a position of the first component.

The following descriptions are only the preferred embodiments of the present disclosure, but are not construed as limiting the scope of the present disclosure. Additionally, orientation or position relationships indicated by terms "vertical", "horizontal", "top", "bottom", "front", "rear", "upper", "lower", and the like, mentioned in the embodiments of the present disclosure are orientation or position relationships shown in the drawings, or orientation or position relationships based on which products are usually placed, are adopted not to indicate or imply that indicated devices or elements must be in specific orientations or structured and operated in specific orientations but only to conveniently describe the present disclosure and simplify the description, and thus should not be understood as a limitation to the present disclosure.

It is further noted that unless otherwise clearly specified and limited, terms such as "mounting", "connection", "link", and "fixing" should be broadly understood. For example, the term "connection" may refer to fixed connection, detachable connection or integrated connection; or direct connection, indirect connection through a medium, or communication between interiors of two elements. For those ordinarily skilled in the art, the specific meaning of the above terms in the present disclosure can be understood according to specific circumstances.

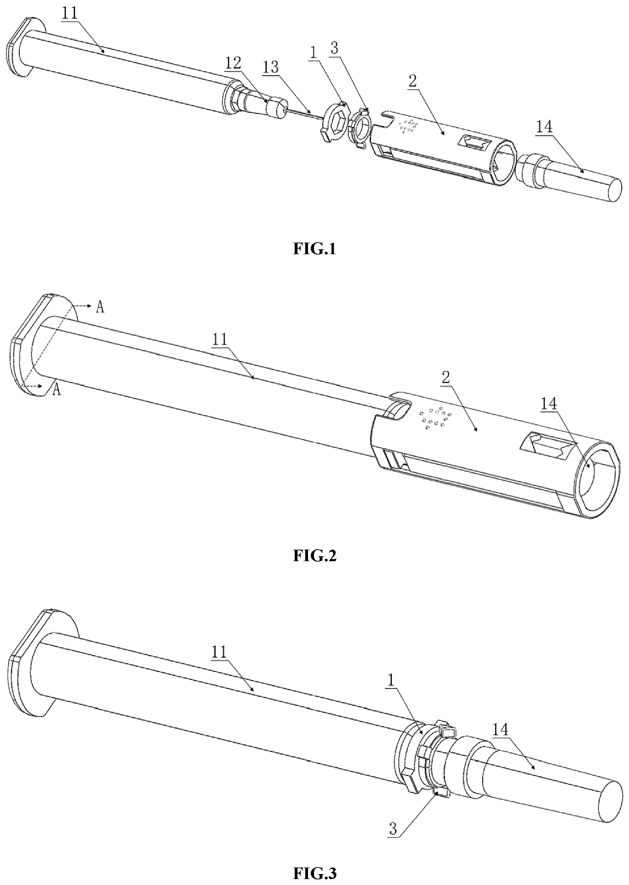

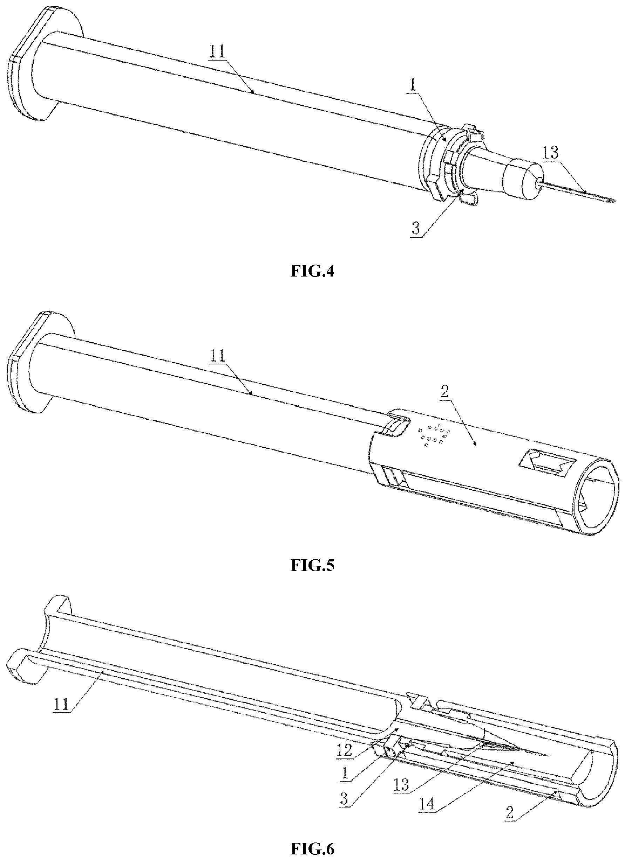

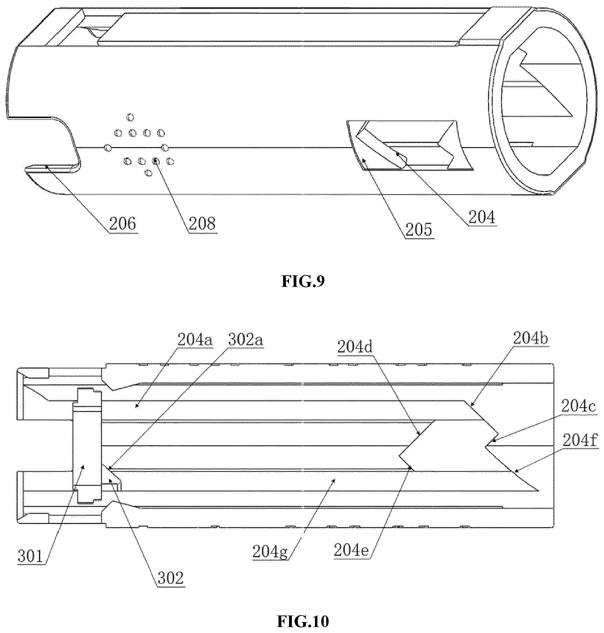

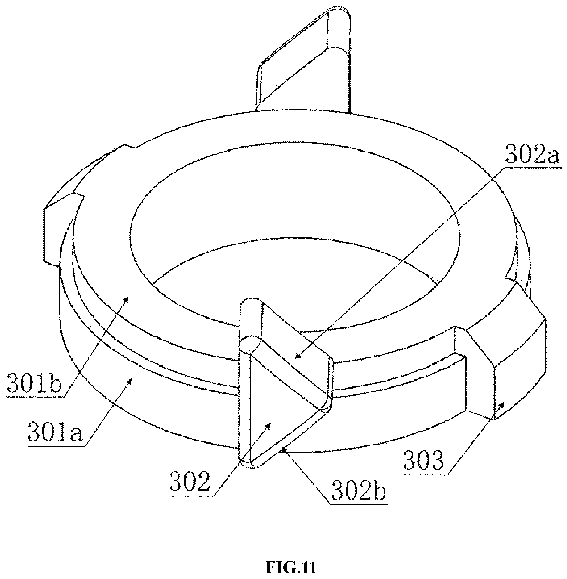

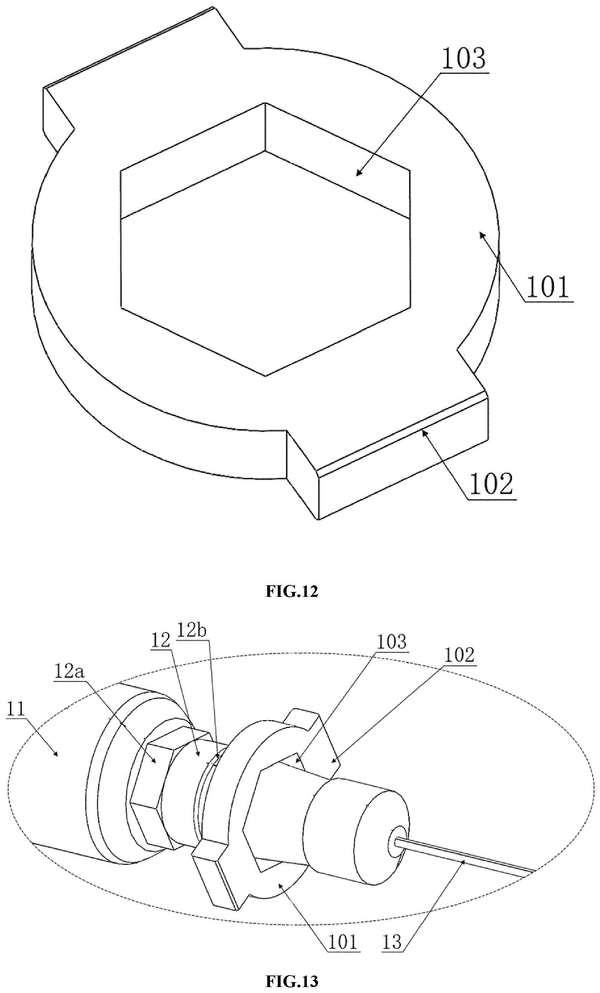

Embodiment: As shown in FIG. 1 to FIG. 13, a needle protection type pre-filled barrel, includes: a barrel 11, a needle mounting tube 12, an injection needle 13, and a sealing sleeve 14, wherein
the needle mounting pipe 12 is arranged on the barrel 11 and used for mounting the injection needle 13, the barrel 11, the needle mounting tube 12, and the injection needle 13 are in a connected state, and the sealing sleeve 14 sleeves the needle mounting tube 12 and is used for protecting the injection needle 13, wherein the needle protection type pre-filled barrel further includes:
a limiting block unit 1, including limiting blocks 102 mounted on the needle mounting tube 12; and
a protective cover unit 2, including a protective cover 201 sleeving part of the barrel 11 and all the needle mounting tube 12 and injection needle 13, limiting ports 202 being formed in the protective cover 201 and used for engaging with the limiting block unit 1.

In this embodiment, the pre-filled barrel is not provided with a push rod and a plunger when leaving the factory, but is directly delivered to a downstream manufacturer for filling medicine, and then the piston is arranged additionally. The barrel 11, the needle mounting tube 12, the injection needle 13, and the sealing sleeve 14 are necessary structures for achieving an injection function of the barrel in the prior art, and after the barrel 11, the needle mounting tube 12, the injection needle 13, and the sealing sleeve 14 are assembled completely, the pre-filled barrel is mounted in the following manner:
1. the limiting blocks 102 are arranged on the needle mounting tube 12. If the limiting blocks 102 are of a movable mounting structure, the limiting blocks 102 are first mounted on the needle mounting tube 12.
2. The protective cover 201 sleeves the barrel 11, and the limiting blocks 102 are allowed to engage into the limiting ports 202.

A use method for the pre-filled barrel is as follows:
1. the protective cover 201 is pulled proximally along the barrel 11 to expose the sealing sleeve 14;
2. the sealing sleeve 14 is removed from the needle mounting tube 12 to expose the injection needle 13;
3. injection is carried out; and
4. after the injection is finished, the protective cover 201 is pushed distally along the barrel 11 till the injection needle 13 is completely covered.

Wherein a width of a protrusion portion of the limiting block 102 is slightly smaller than a width of the limiting port 202, and the protective cover unit 2 moves along the barrel 11 without rotating relative to the barrel 11 when the protrusion portion of the limiting block is inserted into the limiting port 202;

Additionally, friction force exists between the limiting blocks 102 and the protective cover unit 2, such that the protective cover 201 is fixed in position without being manually pushed or pulled, does not slide distally during injection, and does not slide proximally during the protection of the injection needle 13.

Furthermore, a syringe undergoes an inspection process called "visual inspection" before leaving the factory. The "visual inspection" involves visual observation of the barrel to inspect whether the fill-finishing and stoppering of the syringe are up to standard. However, most integrated protective covers on the market affect this process. For example, the Chinese utility model with Publication No. CN214911888U, published on November 30, 2021, has disclosed a syringe needle protection device and a safety syringe, the syringe needle protection device includes an outer protective sleeve, an inner mounting sleeve, and a spring. This sleeve-in-sleeve manner of the inner sleeve and the outer sleeve obstructs the view inside the barrel, resulting in impacting the visual inspection process. The protective cover unit 2 in this embodiment only covers the needle mounting tube 12 and one part of an end of the barrel 11 at a distal end under the condition of not using the barrel, which does not interfere with the visual inspection process.

In order to allow an operator to release hands after the injection, the protective cover 201 may automatically slide in a distal direction, the protective cover unit 2 further includes an energy arm portion 203, the energy arm portion 203 includes an energy arm 203a, one end of which is arranged on an inner side surface of the limiting port 202 at the distal end, and the other end of which has an elastic force directed inward in a radial direction of the protective cover 201.

When the operator pulls the protective cover 201 proximally, the limiting blocks 102 jack the energy arm 203a outward in the radial direction of the protective cover 201, such that the energy arm 203a stores energy, and the protective cover 201 has a tendency to move distally under the action of the energy arm 203a. After the injection is finished, the operator releases both hands, and the energy arm 203a releases energy to act on the limiting blocks 102, such that the protective cover 201 moves distally till the injection needle 13 is completely covered.

In order to lock the protective cover 201 and prevent the protective cover from moving proximally again when the protective cover 201 moves distally till the injection needle 13 is completely covered, the energy arm 203a further has a straight surface 203b for locking at the proximal end for jacking the limiting blocks 102 to lock relative positions between the protective cover 201 and the injection needle 13. In this way, the injection needle 13 is not easily exposed when discarded.

When the straight surface 203b for locking of the energy arm 203a just slides down to engage with the limiting blocks 102, a "click" sound is produced to alert the operator that the protective cover 201 is completely engaged and is ready for discarding.

In order to facilitate the smoother energy storage process of the energy arm 203a during the movement of the protective cover 201 toward the proximal end, further, the energy arm portion 203 further includes a jacking block 203c arranged at the proximal end of the energy arm 203a, when the protective cover 201 moves proximally, the limiting blocks 102 jack the energy arm 203a outward in the radial direction of the protective cover 201, and the jacking block 203c also abuts against an outer wall of the barrel 11, causing the energy arm 203a to experience a force directed outward in the radial direction of the protective cover 201. The combined action of the jacking force and the abut-against force abuts against the energy arm 203a together to support the outward expansion of the energy arm, ensuring a more uniform force bearing and expansion process of the energy arm.

Additionally, the proximal end of the jacking block 203c has an inclined surface 203d for unlocking. When the jacking block 203c passes over the limiting blocks 102, the inclined surface 203d for unlocking can slide over upper ends of the limiting blocks 102, making the passing-over process easier.

In order to allow the jacking block 203c to pass over the limiting blocks 102 without specifically pulling the energy arm 203a outward, when the protective cover 201 is unlocked proximally, further, the energy arm 203a or the jacking block 203c is jacked outward in a radial direction of the barrel 11 when the sealing sleeve 14 sleeves the needle mounting tube 12, such that the straight surface 203b for locking disengages from the limiting blocks 102.

Such an arrangement has the advantage that when the sealing sleeve 14 sleeves the needle mounting tube 12 to protect the injection needle 13, the protective cover 201 can move freely toward the proximal end, and after the sealing sleeve 14 is pulled out of the needle mounting tube 12, the protective cover 201 is reset toward the distal end through the energy arm 203a, the energy arm 203a loses the support of the sealing sleeve 14, and then falls down, such that the straight surface 203b for locking jacks the limiting blocks 102 to complete locking; and during the whole process, the operator can complete the operation by one hand without specifically pulling the energy arm 203a during the unlocking process.

In order to enable the protective cover 201 to be fixed after sliding toward the proximal end, further, the needle protection type pre-filled barrel further includes a rotating limiting unit 3, wherein the rotating limiting unit 3 includes a rotating ring 301 sleeving the needle mounting tube 12 and guide blocks 302 arranged on an outer ring surface of the rotating ring 301, and each of the guide blocks 302 has a distal inclined surface 302a and a proximal inclined surface 302b;
the protective cover unit 2 further includes a guide groove portion 204, and the guide groove portion includes:
an inlet groove 204a, which is formed in an inner ring surface of the protective cover 201 and used for mounting the guide blocks 302;
a first guide surface 204b, which is located at a distal end of the inlet groove 204a, wherein when the protective cover 201 moves toward a proximal end, the first guide surface 204b and the distal inclined surfaces 302a slide in a fit manner, so as to drive the rotating ring 301 to rotate;
a second guide surface 204c, which is connected with the first guide surface 204b and used for jacking the guide blocks 302 to prevent the rotating ring 301 from rotating;
a third guide surface 204d, which is arranged on the inner ring surface of the protective cover 201 and located at a proximal end of the second guide surface 204d, wherein when the protective cover 201 moves toward a distal end, the third guide surface 204d and the proximal inclined surfaces 302b slide in a fit manner, so as to drive the rotating ring 301 to rotate;
a fourth guide surface 204e, which is connected with the third guide surface 204d and used for jacking the guide blocks 302 to prevent the rotating ring 301 from rotating; and
a fifth guide surface 204f, which is arranged on the inner ring surface of the protective cover 201 and located at a distal end of the third guide surface 204d, wherein when the protective cover 201 moves toward the proximal end, the fifth guide surface 204f and the distal inclined surfaces 302a slide in a fit manner, so as to drive the rotating ring 301 to rotate to enter a withdrawal groove 204g formed in the inner ring surface of the protective cover 201.

After the rotation limiting unit 3 and the guide groove portion 204 are arranged, the pre-filled barrel is used as follows:
1. the protective cover 201 is pulled proximally along the barrel 11 to expose the sealing sleeve 14. As a result, the guide blocks 302 enter the inlet groove 204a, and the pulling of the protective cover 201 toward the proximal end along the barrel 11 continues; the distal inclined surfaces 302a of the guide blocks 302 fit and slide along the first guide surface 204b till the guide blocks 302 stop moving upon abutting against the second guide surface 204c, and as a result, the entire sealing sleeve 14 is exposed;
2. the protective cover 201 is released, allowing the protective cover 201 to move distally under the action of the energy arm 203a. As a result, the proximal inclined surfaces 302b of the guide blocks 302 fit and slide along the third guide surface 204d till the guide blocks 302 stop moving upon abutting against the fourth guide surface 204e, and the protective cover 201 is locked;
3. the sealing sleeve 14 is removed from the needle mounting tube 12 to expose the injection needle 13, and a distance where the protective cover 201 moves distally does not affect the removal process of the sealing sleeve 14;
4. injection is carried out; and
5. after the injection is completed, the protective cover 201 is pulled proximally along the barrel 11. As a result, the distal inclined surfaces 302a of the guide blocks 302 fit and slide along the fifth guide surface 204f till the guide blocks 302 either abut against a groove surface of the withdrawal groove 204g or stop moving upon reaching a limit expanded distance of the energy arm 203a. When the protective cover 201 is released, energy from the energy arm 203a is released, ejecting the protective cover 201 toward the distal end. During this process, the guide blocks 302 slide along the withdrawal groove 204g till the straight surface 203b for locking on the energy arm 203a engages with the limiting blocks 102, thus completing the locking of the protective cover 201.

The arrangement of the rotating limiting unit 3 and the guide groove portion 204 has the following advantages:
1. the operator only needs to pull the protective cover 201 proximally to accomplish steps 1-4, and remains completely away from the injection needle 13 throughout the process, thus effectively avoiding the risk of needlestick injury during operation; and
2. the operation of pulling the protective cover 201 in the proximal direction is very simple, necessitating nothing more than the mere exertion of the thumb and index finger to pull the protective cover 201 toward the center of the palm. In this way, even the operator with smaller fingers can operate with ease.

During the actual production process, the specifications of the sealing sleeves for the injection needles are different, and some smaller-sized sealing sleeves are unable to fully jack the energy arm 203a, resulting in the necessity for the operator to disengage the energy arm 203a from the limiting blocks 102 with the other hand in step 1. Furthermore, the rotating limiting unit 3 further includes energy arm jacking blocks 303 arranged on the rotating ring 301. When the guide blocks 302 are located in the inlet groove 204a, the energy arm jacking blocks 303 jack the energy arm 203a or the jacking block 203c outward in the radial direction of the protective cover 201, such that the straight surface 203b for locking disengages from the limiting blocks 102.

In step 1, the guide blocks 302 are located in the inlet groove 204a, where the energy arm jacking blocks 303 just jack the energy arm 203a. In step 4, the guide blocks 302 are located in the withdrawal groove 204g, where the rotating ring 301 rotates by a certain angle, causing the energy arm jacking blocks 303 to become misaligned with the energy arm 203a. This allows the energy arm 203a to engage with the limiting blocks 102, thus achieving the locking function. According to a clever design, the energy arm 203a can be unlocked before the syringe is used based on the positional change of the guide blocks 302.

The protective cover unit 2 further includes a window for demolding 205 that is arranged on the protective cover 201, and the window for demolding 205 exposes a position of the guide groove portion 204 in the inner ring surface of the protective cover 201.

The functions of the window for demolding 205 are as follows:
1. it is convenient to leave a redundancy space for the protective cover unit 2 when separated from a mold at a demolding stage to avoid cracking during the demolding process; and
2. the operator can visually ascertain the engagement state between the guide blocks 302 and the first guide surface 204b, as well as the subsequent engagements with the second guide surface 204c, the third guide surface 204d, the fourth guide surface 204e, and the fifth guide surface 204f, so as to facilitate the validation of alignment of the guide blocks 302 at the appropriate engagement position.

The protective cover unit 2 further includes inclined surfaces 207 for mounting that are arranged at a proximal end of the protective cover 201 and used for jacking the limiting blocks 102 when the protective cover 201 sleeves the needle mounting tube 12, such that the proximal end of the protective cover 201 expands outward in the radial direction.

When sleeving the needle mounting tube 12, the protective cover 201 needs to pass over the limit blocks 102, and the limit blocks 102 are allowed to engage into the limiting ports 202. In this process, the proximal end of the protective cover 201 needs to radially expand to allow the limiting blocks 102 to pass through the protective cover, and the inclined surfaces 207 for mounting are arranged to facilitate the smoother passage of the limiting blocks 102.

Further, the protection cover unit 2 further includes a deformation groove 206 that is formed in the proximal end of the protective cover 201 and provides an expansion space when the proximal end of the protective cover 201 expands outward in the radial direction.

A display surface 203e for printing patterns is arranged on an outer side surface of the energy arm 203a.

The outer side surface of the energy arm 203a is just a plane, where the display surface 203e can be arranged, and the display surface 203e can be used for carving important information such as product models and built-in medicine.

The protective cover unit 2 further includes an indicating member 208 arranged on an outer side surface of the protective cover 201.

The indicating member 208 is a pattern formed by a plurality of small protrusions, which can be used to guide the operator on the direction in which the protective cover 201 should be pulled when in use, thus preventing misuse by the operator who is unclear about how to use the protective cover.

The rotating ring 301 consists of a large rotating ring 301a and a small rotating ring 301b, and when the rotating ring 301 sleeves the needle mounting tube 12, the large rotating ring 301a is located at the proximal end, and the small rotating ring 301b is located at the distal end.

After the jacking block 203c on the energy arm 203a passes over the limiting blocks 102, the jacking block also touches the rotating ring 301, resulting in the risk of jamming. Based on this, the rotating ring 301 is designed into a double-layer ring formed by the large rotating ring 301a and the small rotating ring 301b, the large rotating ring is arranged at the proximal end, and the small rotating ring is arranged at the distal end, such that when the rotating ring 301 jacks the jacking block 203c of the energy arm 203a, and the jacking block 203c passes over the rotating ring 301 along a step formed by the large rotating ring 301a and the small rotating ring 301b, thus avoiding jamming.

The limiting block unit 1 includes a mounting ring 101 sleeving the needle mounting tube 12 and the limiting blocks 102 that are arranged on the mounting ring 101 and are used for engaging with the limiting ports 202.

The limiting block unit 1 is directly arranged on the barrel 11, which may alter the structure of the existing barrel 11 and does not facilitate mass production. The limiting block unit 1 is designed to be mounted in a movable form, such that the appropriately sized mounting ring 101 can be produced to sleeve the needle mounting tube 12 according to the specification of the needle mounting tube 12 on the existing barrel 11, and the mounting of the limiting blocks 102 is completed.

A anti-rotation outer ring surface 12a is arranged on the needle mounting tube 12, and a anti-rotation inner ring surface 103 matched with the anti-rotation outer ring surface 12a is arranged on an inner ring surface of the mounting ring 101 to prevent the limiting blocks 102 from rotating when in use, which would cause the entire protective cover unit 2 to rotate together with the limiting blocks and affect the engagement process.

The needle mounting tube 12 further includes a stop protrusion 12b that engages with the rotating ring 301.

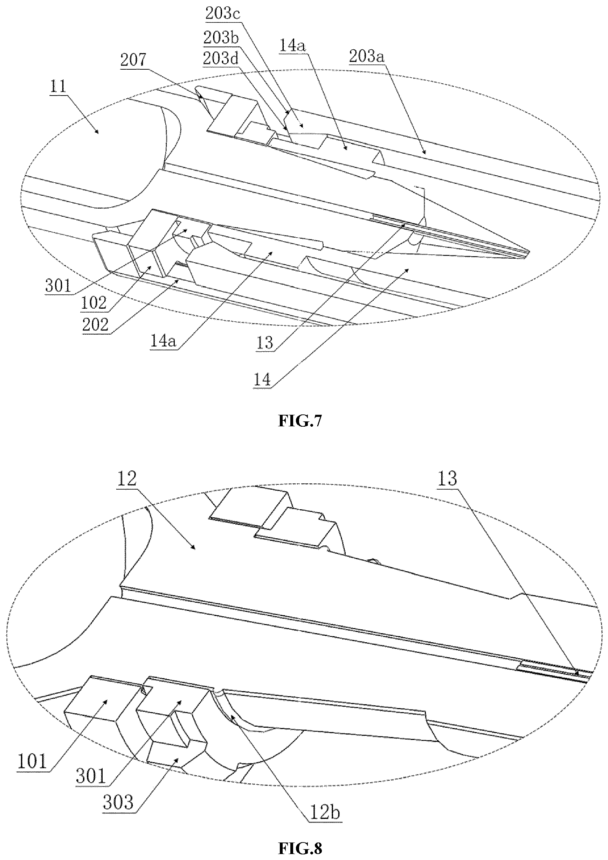

As shown in FIG. 8, when sleeving the needle mounting tube 12, the rotating ring 301 passes over the stop protrusion 12b to engage between the stop protrusion 12b and a distal end surface of the barrel 11, such that the rotating ring 301 can only rotate without making movement in an axial direction of the barrel 11.

An assembly of a needle protection type pre-filled barrel includes a push rod and a plunger, wherein
the push rod is arranged on the plunger; and
the piston is mounted in a barrel 11 and used for pushing liquid medicine out through an injection needle 13 when pushing the push rod toward a distal end.

A mounting method for an assembly, includes the following steps:
S1. a sealing sleeve 14 is axially mounted into a protective cover 201;
S2. the protective cover 201 with the sealing sleeve 14 sleeves a needle mounting tube 12 axially;
S3. a plunger is pushed into a barrel 11; and
S4. a push rod is arranged on the plunger.

A use method for an assembly, includes the following steps:
S1. a protective cover 201 moves axially along a proximal end of a barrel 11, such that a sealing sleeve 14 is exposed;
S2. the sealing sleeve 14 is unlocked, and an injection needle 13 is exposed;
S3, injection is carried out; and
S4, the protective cover 201 axially moves along a distal end of the barrel 11, such that the injection needle 13 is completely covered.

The implementations of the present disclosure are described in detail with reference to the drawings, however, however, the present disclosure is not limited to these. A person ordinarily skilled in the art can also make various modifications within their knowledge range without departing from the purpose of the present disclosure. These modifications are not inventive, and are protected by patent laws as long as they fall within the scope of the claims of the present disclosure.

## Claims

1. A needle protection type pre-filled barrel, comprising: a barrel (11), a needle mounting tube (12), an injection needle (13), and a sealing sleeve (14), wherein
the needle mounting tube (12) is arranged on the barrel (11) and used for mounting the injection needle (13), the barrel (11), the needle mounting tube (12), and the injection needle (13) are in a connected state, and the sealing sleeve (14) sleeves the needle mounting tube (12) and is used for protecting the injection needle (13), the needle protection type pre-filled barrel further comprises:
a limiting block unit (1), comprising limiting blocks (102) mounted on the needle mounting tube (12); and
a protective cover unit (2), comprising a protective cover (201) sleeving part of the barrel (11) and all the needle mounting tube (12) and injection needle (13), limiting ports (202) being formed in the protective cover (201) and used for engaging with the limiting blocks (102);
the protective cover unit (2) further comprises:
an energy arm portion (203), comprising an energy arm (203a), one end of the energy arm is arranged on inner side faces of distal ends of the limiting ports (202), and the other end of the energy arm has an elastic force directed inward in a radial direction of the protective cover (201);
a straight surface (203b) for locking is arranged at a proximal end of the energy arm (203a) and used for jacking the limiting blocks (102) so as to lock relative positions between the protective cover (201) and the injection needle (13);
the energy arm portion (203) further comprises a jacking block (203c) arranged at the proximal end of the energy arm (203a), and an inclined surface (203d) for unlocking is arranged at a proximal end of the jacking block (203c);
when the sealing sleeve (14) sleeves the needle mounting tube (12), the energy arm (203a) or the jacking block (203c) is jacked outward in a radial direction of the barrel (11), such that the straight surface (203b) for locking and the limiting blocks (102) disengage from each other;
further comprising a rotating limiting unit (3), the rotating limiting unit (3) comprises a rotating ring (301) sleeving the needle mounting tube (12) and guide blocks (302) arranged on an outer ring surface of the rotating ring (301), and the guide blocks (302) are provided with distal inclined surfaces (302a) and proximal inclined surfaces (302b);
the protective cover unit (2) further comprises a guide groove portion (204), and the guide groove portion comprises:
an inlet groove (204a) formed in an inner ring surface of the protective cover (201) and used for mounting the guide blocks (302);
a first guide surface (204b) located at a distal end of the inlet groove (204a), when the protective cover (201) moves toward a proximal end, the first guide surface (204b) and the distal inclined surfaces (302a) slide in a fit manner, so as to drive the rotating ring (301) to rotate;
a second guide surface (204c) connected with the first guide surface (204b) and used for jacking the guide blocks (302) to prevent the rotating ring (301) from rotating;
a third guide surface (204d) arranged on the inner ring surface of the protective cover (201) and located at a proximal end of the second guide surface (204d), when the protective cover (201) moves toward a distal end, the third guide surface (204d) and the proximal inclined surfaces (302b) slide in a fit manner, so as to drive the rotating ring (301) to rotate;
a fourth guide surface (204e) connected with the third guide surface (204d) and used for jacking the guide blocks (302) to prevent the rotating ring (301) from rotating; and
a fifth guide surface (204f) arranged on the inner ring surface of the protective cover (201) and located at a distal end of the third guide surface (204d), when the protective cover (201) moves toward the proximal end, the fifth guide surface (204f) and the distal inclined surfaces (302a) slide in a fit manner, so as to drive the rotating ring (301) to rotate to enter a withdrawal groove (204g) formed in the inner ring surface of the protective cover (201);
**characterised in that**
the rotating limiting unit (3) further comprises energy arm jacking blocks (303) arranged on the rotating ring (301), and when the guide blocks (302) are located in the inlet groove (204a), the energy arm jacking blocks (303) jack the energy arm (203a) or the jacking block (203c) outward in the radial direction of the protective cover (201), such that the straight surface (203b) for locking and the limiting blocks (102) disengage from each other.

2. The needle protection type pre-filled barrel according to claim 1, wherein the protective cover unit (2) further comprises a window (205) for demolding formed in the protective cover (201), and the window (205) for demolding exposes a position of the guide groove portion (204) in the inner ring surface of the protective cover (201).

3. The needle protection type pre-filled barrel according to claim 1, wherein the protective cover unit (2) further comprises inclined surfaces (207) for mounting arranged at a proximal end of the protective cover (201) and used for jacking the limiting blocks (102) when the protective cover (201) sleeves the needle mounting tube (12), such that the proximal end of the protective cover (201) expands outward in a radial direction.

4. The needle protection type pre-filled barrel according to claim 1, wherein the protective cover unit (2) further comprises a deformation groove (206) that is formed in a proximal end of the protective cover (201) and used for providing an expansion space when the proximal end of the protective cover (201) expands outward in a radial direction.

5. The needle protection type pre-filled barrel according to claim 1, wherein a display surface (203e) for printing patterns is arranged on an outer side face of the energy arm (203a).

6. The needle protection type pre-filled barrel according to claim 1, wherein the protective cover unit (2) further comprises an indicating member (208) arranged on an outer side face of the protective cover (201).

7. The needle protection type pre-filled barrel according to claim 1, wherein the rotating ring (301) consists of a large rotating ring (301a) and a small rotating ring (301b), and when the rotating ring (301) sleeves the needle mounting tube (12), the large rotating ring (301a) is located at the proximal end, and the small rotating ring (301b) is located at the distal end.

8. The needle protection type pre-filled barrel according to claim 1, wherein the limiting block unit (1) further comprises a mounting ring (101) mounted on the needle mounting tube (12) in a sleeving manner, and the limiting blocks (102) are mounted on the needle mounting tube (12) by being arranged on the mounting ring (101).

9. The needle protection type pre-filled barrel according to claim 8, wherein a anti-rotation outer ring surface (12a) is arranged on the needle mounting tube (12), and a anti-rotation inner ring surface (103) matched with the anti-rotation outer ring surface (12a) is arranged on an inner ring surface of the mounting ring (101).

10. The needle protection type pre-filled barrel according to claim 1, wherein the needle mounting tube (12) further comprises a stop protrusion (12b) used for engaging with the rotating ring (301).

11. An assembly of the needle protection type pre-filled barrel according to claim 1-10, comprising a push rod and a plunger, wherein
the push rod is arranged on the plunger; and
the plunger is mounted in a barrel (11) and used for pushing out liquid medicine through an injection needle (13) when pushing the push rod toward a distal end.

12. A mounting method for the assembly according to claim 11, comprising the following steps:
S1, axially mounting a sealing sleeve (14) into a protective cover (201);
S2, axially sleeving a needle mounting tube (12) with the protective cover (201) with the sealing sleeve (14);
S3, pushing a plunger into a barrel (11); and
S4, arranging a push rod on the plunger.

## Patentansprüche

1. Ein vorgefüllter Zylinder mit Nadelschutz, umfassend: einen Zylinder (11), ein Nadelbefestigungsrohr (12), eine Injektionsnadel (13) und eine Dichtungshülse (14), wobei
das Nadelbefestigungsrohr (12) ist auf dem Zylinder (11) angeordnet und dient zur Aufnahme der Injektionsnadel (13), Zylinder (11), Nadelbefestigungsrohr (12) und Injektionsnadel (13) sind miteinander verbunden, die Dichtungshülse (14) umschließt das Nadelbefestigungsrohr (12) und dient zum Schutz der Injektionsnadel (13), der vorgefüllte Zylinder mit Nadelschutz umfasst ferner:
eine Begrenzungsblockeinheit (1), bestehend aus Begrenzungsblöcken (102), die auf dem Nadelbefestigungsrohr (12) montiert sind; und
eine Schutzabdeckungseinheit (2), bestehend aus einer Schutzabdeckung (201), die einen Teil des Zylinders (11) und das gesamte Nadelbefestigungsrohr (12) sowie die Injektionsnadel (13) umschließt, wobei in der Schutzabdeckung (201) Begrenzungsöffnungen (202) ausgebildet sind, die zum Eingriff mit den Begrenzungsblöcken (102) dienen.
die Schutzabdeckungseinheit (2) umfasst ferner:
ein Energiearmabschnitt (203), bestehend aus einem Energiearm (203a), dessen eines Ende an den inneren Seitenflächen der distalen Enden der Begrenzungsöffnungen (202) angeordnet ist und dessen anderes Ende eine elastische Kraft aufweist, die in radialer Richtung nach innen auf die Schutzabdeckung (201) gerichtet ist;
am proximalen Ende des Energiearms (203a) ist eine gerade Fläche (203b) zum Verriegeln angeordnet, die zum Anheben der Begrenzungsblöcke (102) dient, um die relativen Positionen zwischen der Schutzabdeckung (201) und der Injektionsnadel (13) zu verriegeln;
der Energiearmabschnitt (203) umfasst ferner einen am proximalen Ende des Energiearms (203a) angeordneten Hebeblock (203c) und eine geneigte Fläche (203d) zum Entriegeln, die am proximalen Ende des Hebeblocks (203c) angeordnet ist;
wenn die Dichtungshülse (14) das Nadelbefestigungsrohr (12) umschließt, wird der Energiearm (203a) oder der Hebeblock (203c) in radialer Richtung des Zylinders (11) nach außen gedrückt, sodass sich die gerade Fläche (203b) zur Verriegelung und die Begrenzungsblöcke (102) voneinander lösen;
ferner umfassend eine drehbare Begrenzungseinheit (3), wobei die drehbare Begrenzungseinheit (3) einen drehbaren Ring (301) umfasst, der das Nadelmontagerohr (12) umschließt, und auf einer Außenringfläche des drehbaren Rings (301) angeordnete Führungsblöcke (302), wobei die Führungsblöcke (302) mit distalen geneigten Flächen (302a) und proximalen geneigten Flächen (302b) versehen sind;
die Schutzabdeckungseinheit (2) umfasst ferner einen Führungsnutabschnitt (204), und der Führungsnutabschnitt umfasst:
eine in einer inneren Ringfläche der Schutzabdeckung (201) ausgebildete Einlassnut (204a) zur Montage der Führungsblöcke (302);
eine erste Führungsfläche (204b) am distalen Ende der Einlassnut (204a), wobei beim Bewegen der Schutzabdeckung (201) in Richtung ihres proximalen Endes die erste Führungsfläche (204b) und die distalen geneigten Flächen (302a) passgenau ineinander gleiten, um den Drehring (301) in Rotation zu versetzen;
eine zweite Führungsfläche (204c), die mit der ersten Führungsfläche (204b) verbunden ist und zum Anheben der Führungsblöcke (302) dient, um zu verhindern, dass sich der Drehring (301) dreht;
eine dritte Führungsfläche (204d), die auf der inneren Ringfläche der Schutzabdeckung (201) angeordnet und an einem proximalen Ende der zweiten Führungsfläche (204d) angeordnet ist, wobei beim Bewegen der Schutzabdeckung (201) in Richtung eines distalen Endes die dritte Führungsfläche (204d) und die proximalen geneigten Flächen (302b) passgenau gleiten, um den rotierenden Ring (301) in Rotation zu versetzen;
eine vierte Führungsfläche (204e), die mit der dritten Führungsfläche (204d) verbunden ist und zum Anheben der Führungsblöcke (302) dient, um ein Verdrehen des Drehrings (301) zu verhindern; und
eine fünfte Führungsfläche (204f), die auf der inneren Ringfläche der Schutzabdeckung (201) angeordnet und an einem distalen Ende der dritten Führungsfläche (204d) angeordnet ist, wobei sich die Schutzabdeckung (201) in Richtung des proximalen Endes bewegt, gleiten die fünfte Führungsfläche (204f) und die distalen geneigten Flächen (302a) passgenau, um den Drehring (301) in eine in der inneren Ringfläche der Schutzabdeckung (201) ausgebildete Auszugsnut (204g) zu drehen;
**gekennzeichnet durch**
die rotierende Begrenzungseinheit (3) umfasst ferner auf dem Drehring (301) angeordnete Energiearm-Hebeblöcke (303), und wenn sich die Führungsblöcke (302) in der Einlassnut (204a) befinden, heben die Energiearm-Hebeblöcke (303) den Energiearm (203a) oder den Hebeblock (203c) in radialer Richtung der Schutzabdeckung (201) nach außen, sodass die gerade Fläche (203b) zur Verriegelung und die Begrenzungsblöcke (102) voneinander entkoppeln.

2. Der vorgefüllte Zylinder mit Nadelschutz nach Anspruch 1, wobei die Schutzabdeckungseinheit (2) ferner ein in der Schutzabdeckung (201) ausgebildetes Fenster (205) zum Entformen aufweist und das Fenster (205) zum Entformen eine Position des Führungsnutabschnitts (204) in der Innenringoberfläche der Schutzabdeckung (201) freilegt.

3. Der vorgefüllte Zylinder mit Nadelschutz nach Anspruch 1, wobei die Schutzabdeckungseinheit (2) ferner geneigte Flächen (207) zur Montage aufweist, die an einem proximalen Ende der Schutzabdeckung (201) angeordnet sind und zum Anheben der Begrenzungsblöcke (102) dienen, wenn die Schutzabdeckung (201) das Nadelmontagerohr (12) umschließt, sodass sich das proximale Ende der Schutzabdeckung (201) radial nach außen ausdehnt.

4. Der vorgefüllte Zylinder mit Nadelschutz nach Anspruch 1, wobei die Schutzabdeckungseinheit (2) ferner eine Deformationsnut (206) aufweist, die in einem proximalen Ende der Schutzabdeckung (201) ausgebildet ist und dazu dient, einen Expansionsraum bereitzustellen, wenn sich das proximale Ende der Schutzabdeckung (201) radial nach außen ausdehnt.

5. Der vorgefüllte Lauf mit Nadelschutz nach Anspruch 1, wobei auf der Außenseite des Energiearms (203a) eine Anzeigefläche (203e) zum Drucken von Mustern angeordnet ist.

6. Der vorgefüllte Zylinder mit Nadelschutz nach Anspruch 1, wobei die Schutzabdeckungseinheit (2) ferner ein Anzeigeelement (208) umfasst, das auf einer Außenseite der Schutzabdeckung (201) angeordnet ist.

7. Der vorgefüllte Zylinder mit Nadelschutz nach Anspruch 1, wobei der Drehring (301) aus einem großen Drehring (301a) und einem kleinen Drehring (301b) besteht und sich beim Umschließen des Nadelbefestigungsrohrs (12) durch den Drehring (301) der große Drehring (301a) am proximalen Ende und der kleine Drehring (301b) am distalen Ende befindet.

8. Der vorgefüllte Zylinder mit Nadelschutz nach Anspruch 1, wobei die Begrenzungsblockeinheit (1) ferner einen auf dem Nadelbefestigungsrohr (12) hülsenförmig montierten Befestigungsring (101) umfasst und die Begrenzungsblöcke (102) auf dem Nadelbefestigungsrohr (12) durch Anordnung auf dem Befestigungsring (101) montiert sind.

9. Der vorgefüllte Zylinder mit Nadelschutz nach Anspruch 8, wobei auf dem Nadelbefestigungsrohr (12) eine drehsichere Außenringfläche (12a) angeordnet ist und auf einer Innenringfläche des Befestigungsrings (101) eine mit der drehsicheren Außenringfläche (12a) übereinstimmende drehsichere Innenringfläche (103) angeordnet ist.

10. Der vorgefüllte Zylinder mit Nadelschutz nach Anspruch 1, wobei das Nadelbefestigungsrohr (12) ferner einen Anschlagvorsprung (12b) aufweist, der zum Eingriff mit dem Drehring (301) dient.

11. Eine Anordnung mit vorgefülltem Zylinder vom Nadelschutztyp nach Anspruch 1-10, umfassend eine Schubstange und einen Kolben, wobei
die Schubstange ist am Kolben angeordnet; und
der Kolben ist in einer Nadel (11) montiert und dient dazu, flüssige Medizin durch eine Injektionsnadel (13) herauszudrücken, indem die Schubstange in Richtung des distalen Endes gedrückt wird.

12. Montageverfahren für die Baugruppe nach Anspruch 11, umfassend die folgenden Schritte:
S1, axiale Montage einer Dichtungshülse (14) in eine Schutzabdeckung (201);
S2, axiales Ummanteln eines Nadelbefestigungsrohres (12) mit der Schutzkappe (201) und der Dichtungshülse (14);
S3, Drücken eines Kolbens in einen Zylinder (11); und
S4, Anbringen einer Schubstange am Kolben.

## Revendications

1. Un corps prérempli de type protection d'aiguille, comprenant: un corps (11), un tube de montage d'aiguille (12), une aiguille d'injection (13) et un manchon d'étanchéité (14), dans lequel
le tube de montage de l'aiguille (12) est disposé sur le corps (11) et sert à monter l'aiguille d'injection (13), le corps (11), le tube de montage de l'aiguille (12) et l'aiguille d'injection (13) sont solidaires, le manchon d'étanchéité (14) enveloppe le tube de montage de l'aiguille (12) et protège l'aiguille d'injection (13), le corps prérempli avec protection d'aiguille comprend en outre:
une unité de bloc de limitation (1), comprenant des blocs de limitation (102) montés sur le tube de montage de l'aiguille (12); et
une unité de couverture de protection (2), comprenant une couverture de protection (201) recouvrant une partie du canon (11) et l'ensemble du tube de montage de l'aiguille (12) et de l'aiguille d'injection (13), des ports de limitation (202) étant formés dans la couverture de protection (201) et utilisés pour s'engager avec les blocs de limitation (102),
l'unité de couverture de protection (2) comprend en outre:
une partie de bras d'énergie (203), comprenant un bras d'énergie (203a), une extrémité du bras d'énergie est disposée sur les faces latérales internes des extrémités distales des ports de limitation (202), et l'autre extrémité du bras d'énergie a une force élastique dirigée vers l'intérieur dans une direction radiale du couvercle de protection (201);
une surface droite (203b) pour le verrouillage est disposée à une extrémité proximale du bras d'énergie (203a) et utilisée pour lever les blocs de limitation (102) afin de verrouiller les positions relatives entre le couvercle de protection (201) et l'aiguille d'injection (13);
la partie bras d'énergie (203) comprend en outre un bloc de levage (203c) disposé à l'extrémité proximale du bras d'énergie (203a), et une surface inclinée (203d) pour le déverrouillage est disposée à une extrémité proximale du bloc de levage (203c);
lorsque le manchon d'étanchéité (14) enveloppe le tube de montage de l'aiguille (12), le bras d'énergie (203a) ou le bloc de levage (203c) est poussé vers l'extérieur dans une direction radiale du canon (11), de sorte que la surface droite (203b) pour le verrouillage et les blocs de limitation (102) se désengagent l'un de l'autre;
comprenant en outre une unité de limitation rotative (3), l'unité de limitation rotative (3) comprend un anneau rotatif (301) enveloppant le tube de montage de l'aiguille (12) et des blocs de guidage (302) disposés sur une surface extérieure de l'anneau rotatif (301), et les blocs de guidage (302) sont dotés de surfaces inclinées distales (302a) et de surfaces inclinées proximales (302b);
l'unité de couvercle de protection (2) comprend en outre une partie de rainure de guidage (204), et la partie de rainure de guidage comprend:
une rainure d'entrée (204a) formée dans une surface annulaire intérieure du couvercle de protection (201) et utilisée pour monter les blocs de guidage (302);
une première surface de guidage (204b) située à une extrémité distale de la rainure d'entrée (204a), dans laquelle lorsque le couvercle de protection (201) se déplace vers une extrémité proximale, la première surface de guidage (204b) et les surfaces inclinées distales (302a) glissent de manière ajustée, de façon à entraîner la rotation de l'anneau rotatif (301);
une seconde surface de guidage (204c) reliée à la première surface de guidage (204b) et utilisée pour lever les blocs de guidage (302) afin d'empêcher la bague rotative (301) de tourner;
une troisième surface de guidage (204d) disposée sur la surface annulaire intérieure du couvercle de protection (201) et située à une extrémité proximale de la deuxième surface de guidage (204d), dans laquelle lorsque le couvercle de protection (201) se déplace vers une extrémité distale, la troisième surface de guidage (204d) et les surfaces inclinées proximales (302b) glissent de manière ajustée, de façon à entraîner la rotation de l'anneau rotatif (301);
une quatrième surface de guidage (204e) reliée à la troisième surface de guidage (204d) et utilisée pour lever les blocs de guidage (302) afin d'empêcher la rotation de l'anneau rotatif (301); et
une cinquième surface de guidage (204f) disposée sur la surface annulaire intérieure du couvercle de protection (201) et située à une extrémité distale de la troisième surface de guidage (204d), lorsque le couvercle de protection (201) se déplace vers l'extrémité proximale, la cinquième surface de guidage (204f) et les surfaces inclinées distales (302a) glissent de manière ajustée, de façon à entraîner l'anneau rotatif (301) à tourner pour entrer dans une rainure de retrait (204g) formée dans la surface annulaire intérieure du couvercle de protection (201);
**caractérisé en ce que**
l'unité de limitation rotative (3) comprend en outre des blocs de levage de bras d'énergie (303) disposés sur l'anneau rotatif (301), et lorsque les blocs de guidage (302) sont situés dans la rainure d'entrée (204a), les blocs de levage de bras d'énergie (303) soulèvent le bras d'énergie (203a) ou le bloc de levage (203c) vers l'extérieur dans la direction radiale du couvercle de protection (201), de sorte que la surface droite (203b) de verrouillage et les blocs de limitation (102) se désengagent l'un de l'autre.

2. Le corps pré-rempli de type protection d'aiguille selon la revendication 1, dans lequel l'unité de couvercle de protection (2) comprend en outre une fenêtre (205) pour le démoulage formée dans le couvercle de protection (201), et la fenêtre (205) pour le démoulage expose une position de la partie de la rainure de guidage (204) dans la surface annulaire intérieure du couvercle de protection (201).

3. Le canon pré-rempli de type protection d'aiguille selon la revendication 1, dans lequel l'unité de couvercle de protection (2) comprend en outre des surfaces inclinées (207) pour le montage disposées à une extrémité proximale du couvercle de protection (201) et utilisées pour soulever les blocs de limitation (102) lorsque le couvercle de protection (201) enveloppe le tube de montage de l'aiguille (12), de sorte que l'extrémité proximale du couvercle de protection (201) s'étend vers l'extérieur dans une direction radiale.

4. Le canon pré-rempli de type protection d'aiguille selon la revendication 1, dans lequel l'unité de couverture de protection (2) comprend en outre une rainure de déformation (206) formée à une extrémité proximale de la couverture de protection (201) et utilisée pour fournir un espace d'expansion lorsque l'extrémité proximale de la couverture de protection (201) s'étend vers l'extérieur dans une direction radiale.

5. Le canon pré-rempli de type protection d'aiguille selon la revendication 1, dans lequel une surface d'affichage (203e) pour l'impression de motifs est disposée sur une face latérale extérieure du bras d'énergie (203a).

6. Le corps pré-rempli de type protection d'aiguille selon la revendication 1, dans lequel l'unité de couvercle de protection (2) comprend en outre un élément indicateur (208) disposé sur une face latérale extérieure du couvercle de protection (201).

7. Le corps pré-rempli de type protection d'aiguille selon la revendication 1, dans lequel l'anneau rotatif (301) est constitué d'un grand anneau rotatif (301a) et d'un petit anneau rotatif (301b), et lorsque l'anneau rotatif (301) enveloppe le tube de montage de l'aiguille (12), le grand anneau rotatif (301a) est situé à l'extrémité proximale et le petit anneau rotatif (301b) est situé à l'extrémité distale.

8. Le corps pré-rempli de type protection d'aiguille selon la revendication 1, dans lequel l'unité de bloc de limitation (1) comprend en outre un anneau de montage (101) monté sur le tube de montage de l'aiguille (12) de manière emboîtée, et les blocs de limitation (102) sont montés sur le tube de montage de l'aiguille (12) en étant disposés sur l'anneau de montage (101).

9. Le corps pré-rempli de type protection d'aiguille selon la revendication 8, dans lequel une surface annulaire extérieure anti-rotation (12a) est disposée sur le tube de montage de l'aiguille (12), et une surface annulaire intérieure anti-rotation (103) assortie à la surface annulaire extérieure anti-rotation (12a) est disposée sur une surface annulaire intérieure de l'anneau de montage (101).

10. Le corps pré-rempli de type protection d'aiguille selon la revendication 1, dans lequel le tube de montage de l'aiguille (12) comprend en outre une butée (12b) utilisée pour s'engager avec la bague rotative (301).

11. Ensemble de cylindre pré-rempli de type protection d'aiguille selon la revendication 1-10, comprenant une tige de poussée et un piston, dans lequel
la tige de poussée est disposée sur le piston; et
le piston est monté dans une aiguille (11) et est utilisé pour pousser le médicament liquide à travers une aiguille d'injection (13) en poussant la tige de poussée vers une extrémité distale.

12. Procédé de montage de l'ensemble selon la revendication 11, comprenant les étapes suivantes:
S1, montage axial d'un manchon d'étanchéité (14) dans un couvercle de protection (201);
S2, manchonnant axialement un tube de montage d'aiguille (12) avec le couvercle de protection (201) avec le manchon d'étanchéité (14);
S3, poussant un piston dans un barillet (11); et
S4, en disposant une tige de poussée sur le piston.
